## Europäisches Patentamt

## European Patent Office

## Office européen des brevets

(11) Publication number: **0 145 492**
**A2**

(12) # EUROPEAN PATENT APPLICATION

(21) Application number: **84308678.6**

(22) Date of filing: **13.12.84**

(51) Int. Cl.⁴: **A 61 F 2/08**

(30) Priority: **15.12.83 GB 8333432**
**29.02.84 GB 8405213**

(43) Date of publication of application: **19.06.85**
**Bulletin 85/25**

(84) Designated Contracting States: **AT BE CH DE FR GB IT**
**LI LU NL SE**

(71) Applicant: **A.W.SHOWELL (SUGICRAFT) LIMITED,**
**Britten Street, Redditch Worcs B97 6HF (GB)**

(72) Inventor: **Strover, Angus Everett, The School House,**
**Belbroughton, Worcs. (GB)**
Inventor: **Showell, Anthony Dugard, Corner House,**
**Belbroughton, Worcs. (GB)**

(74) Representative: **Hulse, Thomas Arnold et al, Hulse & Co.**
**Cavendish Buildings West Street, Sheffield S1 1ZZ (GB)**

(54) **Replacements for ligaments and tendons.**

(57) A surgical element for use as a replacement or augmentation for a damaged ligament or tendon comprises a multiplicity of flexible filaments (e.g., carbon fibres) held together in substantially parallel array, characterised in that these filaments for a core (11) with braided sheathing (12) over at least one portion. The sheathing filaments may have a more open weave over an intermediate portion of the core, or not braided over a «window» (16), or omitted completely from an intermediate portion, for encouraging penetration and ingrowth of healing tissue between the core filaments. Tails (13) are formed into eyes (18) by looping and secured to bones (19) by attachment devices (20), with the «window» located where it is desired to encourage the ingrowth of healing tissue.

## REPLACEMENTS FOR LIGAMENTS AND TENDONS

This invention relates to surgical elements for use, for example, as replacements for ligaments and tendons, or in augmentation of ruptured ligaments and tendons.

It is known to replace damaged ligaments and tendons, and to augment damaged ligaments and tendons with artificial elements consisting of a multiplicity of fine filaments between which healing tissue can penetrate.

Thus it is known to form a prosthesis, replacement or repair element consisting of a multiplicity of filaments. However, even those made of standard implantable materials have disadvantages in that they tend to break down over a period of time because of stressing and in that they may present problems in anchoring.

It is therefore also known (see GB-PS 1 602 834) to form a prothesis or replacement element for a ligament or tendon consisting of a multiplicity of fine flexible substantially pure carbon fibres, which are inter-woven, inter-twined, or otherwise held together in a generally parallel arrangement, to consitute a coherent multi-fibre cord, and including means at the end of the cord to prevent the cord unravelling at the ends and also to assist in threading the cord through small holes in bones, for example. However, this form of cord is liable to failure through its inability to withstand friction particularly when in contact with rough, abrasive surfaces or sharp edges, which latter may also cause fracture of the carbon fibres if bent sharply over them. The carbon fibres may be individually treated, e.g. resinated, and the edges of the holes in bones may be rounded off or radiused. These measures have been shown to reduce the problem, but do not eliminate it.

The object of the invention is to provide a surgical element for use as a prosthesis, augmentation, replacement or repair element for a ligament or tendon, which does not suffer from the above-mentioned disadvantages.

According to the present invention, a surgical element comprises a core consisting of a multiplicity of substantially parallel flexible filaments of at least one standard implantable grade material, and braided sheathing over at least one portion of the core and consisting of filaments of at least one standard implantable grade material.

The core filaments provide the matrix for the ingrowth of the healing tissue and may contribute to the ultimate tensile strength of the prothesis, while the sheathing protects the core filaments from friction and sharp bending, especially when the core filaments are fine carbon fibres. The material of either the core or sheathing or both may be biologically degradable or absorbable. However, the core filaments may be formed alternatively of bioreactive material such as polymer.

The braiding may be such that healing tissue can penetrate between the sheathing filaments at any position along the length of the prosthesis and also between the core filaments, or the sheathing filaments may extend in more open weave over an intermediate portion of the core, to encourage penetration of healing tissue into that portion. Preferably, however, the sheathing filaments are not braided over at least one intermediate portion of the core, to form at least one "window" in the sheathing encouraging penetration and ingrowth of healing tissue between the core filaments, which "window"in use is located where it is desired to encourage the ingrowth of healing tissue. Thus, the sheathing filaments may extend across the "window" in generally parallel arrangement to unite the braiding at the ends of the "window".

The tensions in the sheathing filaments may be such that they are "prestressed" relative to the core filaments, whereby the core filaments will not normally be in tension, which is advantageous whilst healing tissue is growing into the core.

Alternatively, the sheathing may be completely omitted over an intermediate portion of the core in which case the tightening effect on pulling either end portion of braided sheathing ensures that the end portions grip the core filaments. Such discontinuous sheathing is advantageous from a biological viewpoint, because the completely exposed portion of the core is more readily accessible to healing tissue, which furthermore is not liable to differential stress from differing core and sheathing filaments.

Some end filaments, of the core and/or the sheathing, on one or both ends of the surgical element, may be formed into an eye (or eyes), by looping, for use in securing to bone by means of an attachment device, e.g. a screw, or a button or anchoring device such as described in GB-PS 2 084 468A.

The core filaments may be divided into two or more "trunks", each of which has its own braided sheathing and preferably its own "window". Braided sheathing at one end of the surgical element (or at the end of one - or more - of its trunks) may extend appreciably beyond the ends of the core filaments, to afford a highly flexible non-cored "tail" (or "tails" if the non-cored end is divided into "branches") suitable for knotting after passing through a hole (or holes) in bone or for looping round an anchoring device (e.g. as in the aforesaid GB-PS 2 084 468A); and the or each "tail" may be provided with a plastics sleeve to assist insertion and which may thereafter be cut.off.

A bioabsorbable and biocompatible coating is preferably applied to the surgical element to protect the filaments during surgical implantation and to act as a lubricant to ease its passage through the bone and tissue during the operative procedure.

A number of embodiments of the invention will now be described, by way of example only, with reference to the accompanying drawings, in which:-

Figure 1 is an elevation of one form of surgical element in accordance with the invention;

Figure 2 is an enlarged diagrammatic cross-section on the line II-II of Figure 1;

Figure 3 corresponds to Figure 1 but shows another embodiment in accordance with the invention;

Figure 4 is an enlarged diagrammatic cross-section on the line IV-IV of Figure 3;

Figure 5 is a fragmentary perspective view illustrating two surgical elements as in Figure 3 in use at a joint between two bones; and

Figures 6 and 7 correspond to Figures 1 and 3 but show further embodiments in accordance with the invention.

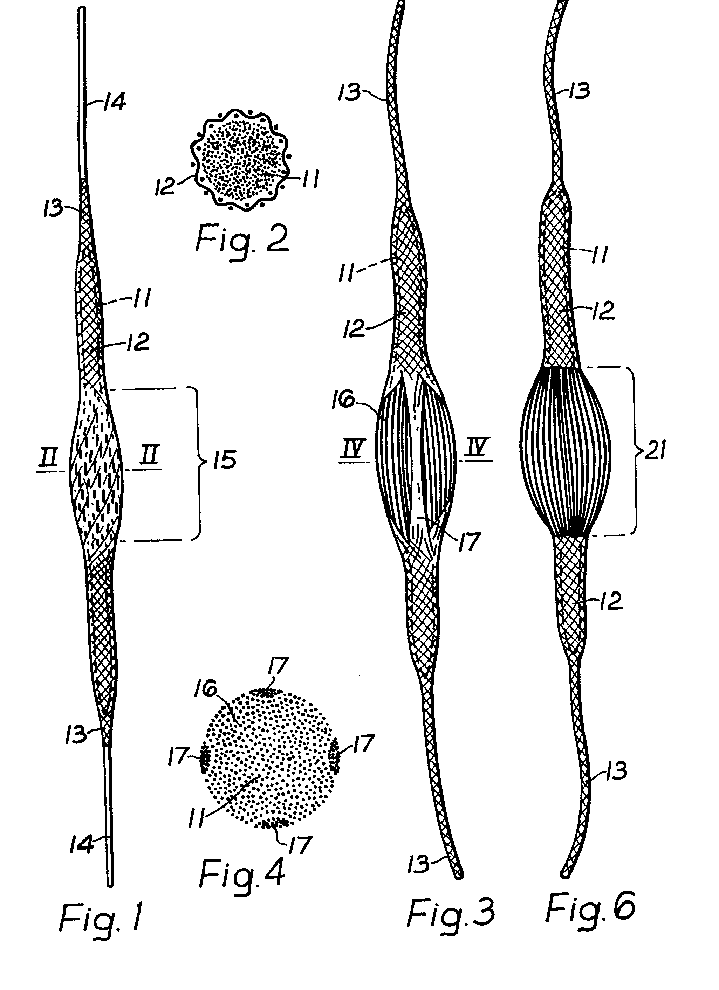

In Figures 1 and 2 a surgical element comprises a core 11 consisting of a multiplicity of substantially parallel flexible filaments of carbon fibre (or other standard implantable grade material), and braided sheathing 12 consisting of filaments of standard implantable grade polymer extending over the entire core and beyond each end of the core filaments to form highly flexible non-cored "tails" 13, which are each shown provided with a plastics sleeve 14 to aid insertion through holes in bones. The sheathing filaments 12 extend in more open weave over an intermedite portion 15 of the core 11, to encourage penetration of healing tissue into that portion. After insertion of the "tails" 13 through holes in bones the sleeves 14 can be cut off, leaving the tails ready to be secured to the bones, by means of attachment devices, e.g. screws, or buttons or anchoring devices of the type described in the aforesaid GB-PS 2 084 468A.

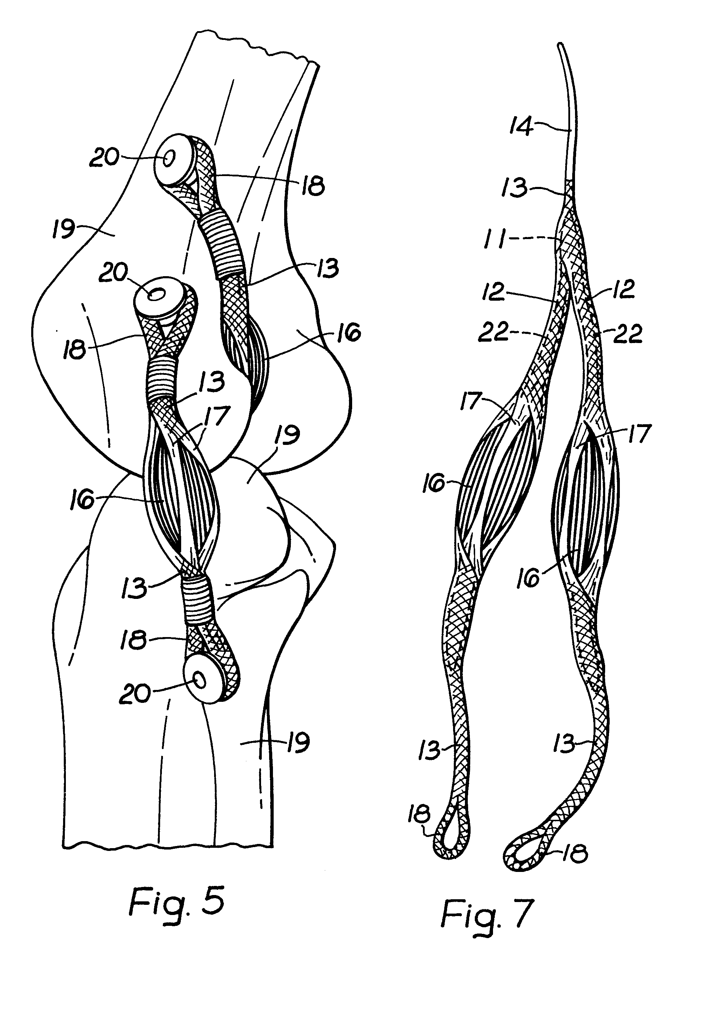

In Figures 3 and 4 a surgical element comprises a core 11 of carbon fibres, and braided sheathing 12 of polymer filaments, but the sheathing filaments are not braided over

- 8 -

0145492

an intermediate portion of the core, to form a "window" 16 in the shething for encouraging penetration and ingrowth of healing tissue between the core filaments. The sheathing filaments extend across the "window" 16 in generally parallel arrangement 17 to unite the braiding 12 at the ends of the "window", and the sheathing filaments may be "prestressed" relative to the core filaments 11, whereby the core filaments will not normally be in tension. The sheathing filaments 12 again extend beyond each end of the core filaments 11 to form highly flexible non-cored "tails" 13, but in this case - as shown in Figure 5 - the "tails" are formed into eyes 18 by looping and secured to bones 19 by means of attachment devices 20, two such surgical elements being shown in Figure 5 with their "widows" located where it is desired to encourage the ingrowth of healing tissue.

In Figure 6 a surgical element comprises a core 11 of carbon fibres, and braided sheathing 12 of polymer filaments, but the latter is completely omitted over an intermediate portion 21 of the core, in which case the tightening effect on pulling either end portion of braided sheathing by its "tail" 13 ensures that the end portions grip the core filaments. Obviously, "prestressing" of the sheathing filaments relative to the core filaments is not possible in this case. Such discontinuous sheathing is, however, advantageous from a biological viewpoint, because the completely exposed portion 21 of the core is more readily accessible to healing tissue, which furthermore is not liable to differential stress from differing core and sheathing filaments.

In Figure 7 a surgical element comprises a core 11 of carbon fibres divided into two "trunks" 22 each of which has its own braided sheathing 12 and its own "window" 16. Each of the "tails" 13 of the sheathed "trunks" is shown formed into an eye 18 by looping, while the common "tail" 13 at the other end is shown provided with a plastics sleeve 14.

CLAIMS

1. A surgical element having a multiplicity of flexible filaments of at least one standard implantable grade material held together in array, characterised in that these filaments constitute a core (11), and in that braided sheathing (12) is provided over at least one portion of the core and consists of filaments of at least one standard implantable grade material.

2. A surgical element as in Claim 1, characterised in that the material of the core (11) and/or sheathing (12) is biologically degradable or absorbable.

3. A surgical element as in Claim 1, characterised in that the core filaments (11) are formed of bioreactive material.

4. A surgical element as in any one of Claims 1 to 3, characterised in that the sheathing filaments (12) extend in more open weave over an intermediate portion (15) of the core (11), to encourage penetration of healing tissue into that portion.

5. A surgical element as in any one of Claims 1 to 3, characterised in that the sheathing filaments (12) are not braided over at least one intermediate portion of the core (11), to form at least one "window" (16) in the sheathing for encouraging penetration and ingrowth of healing tissue between the core filaments.

6. A surgical element as in Claim 5, characterised in that the sheathing filaments (12) extend across the "window" (16) in generally parallel arrangement to unite the braiding at the ends of the "window".

7. A surgical element as in any one of Claims 4 to 6, characterised in that the tensions in the sheathing filaments (12) are such that they are "prestressed" relative to the core filaments (11), whereby the core filaments will not normally be in tension.

8. A surgical element as in any one of Claims 1 to 3, characterised in that the sheathing (12)is completely omitted over an intermediate portion (21) of the core (11).

9. A surgical element as in any one of Claims 1 to 8, characterised in that some end filaments (12) are formed into an eye (18) by looping.

10. A surgical element as in any one of Claims 1 to 9, characterised in that the core filaments (12) are divided into "trunks" (22) each of which has its own braided sheathing (12).

11. A surgical element as in any one of Claims 1 to 10, characterised in that braided sheathing (12) at one end extends appreciably beyond the ends of the core filaments (11), to form a highly flexible non-cored "tail" (13).

12. A surgical element as in Claim 11, characterised in that the "tail" (13) is provided with a plastics sleeve (14) to aid insertion.

13. A surgical element as in any one of Claims 1 to 12, wherein the core filaments are carbon fibres.

14. A surgical element as in any one of Claims 1 to 12, characterised in that the core filaments (11) are formed of at least one standard implantable grade polymer.

15. A surgical element as in any one of Claims 1 to 14, characterised in that a bioabsorbable and biocompatible coating is applied to protect the filaments (11,12) during surgical implantation and to act as a lubricant to ease its passage through the bone and tissue during the operative procedure.

Fig. 2

Fig. 4

Fig. 1

Fig. 3

Fig. 6

0145492

0145492

Fig. 5

Fig. 7